# EUROPEAN PATENT SPECIFICATION

(11) **EP 3 307 082 B1**
(45) Date of publication and mention of the grant of the patent: **31.08.2022**
(21) Application number: 16729838.9
(22) Date of filing: 08.06.2016
(51) Int. Cl.: A23F 3/16, A23P 10/30, A23L 33/21, A61P 3/04, A23L 2/52

(54) **COMPOSITION FOR WEIGHT REDUCTION COMPRISING A BEVERAGE AND BEADS**
ZUSAMMENSETZUNG MIT EINEM GETRÄNK UND KUEGELCHEN ZUR GEWICHTSVERMINDERING
COMPOSITION POUR LA RÉDUCTION DE POIDS COMPRENANT UNE BOISSON ET DES BILLES

(30) Priority: 11.06.2015 EP 15171600
(43) Date of publication of application: 18.04.2018
(73) Proprietor: System Biologie AG, 8832 Wollerau (CH)
(72) Inventor: SCHMID, Juerg, 8832 Wollerau (CH); HUBER, Johannes, 1130 Wien (AT)
(74) Representative: Banse & Steglich Patentanwälte PartmbB
(86) International application number: PCT/EP2016/062971
(87) International publication number: WO 2016/198424

(56) References cited:
- EP-A1- 1 629 722
- EP-B1- 1 629 722
- CN-A- 101 658 221
- CN-A- 103 891 935
- CN-A- 104 247 801
- US-A- 4 233 325
- US-A1- 2005 255 201
- US-A1- 2010 330 189
- US-B1- 6 196 406
- DATABASE GNPD [Online] MINTEL; 18 July 2002 (2002-07-18), anonymous: "Bubble & Pearl Drink Mix", XP055685208, retrieved from www.gnpd.com Database accession no. 10113347

## Description

The invention relates to a composition for oral administration comprising a beverage and beads distributed in the beverage. The invention also relates to uses for reducing weight of an individual.

### State of the art

Overweight and obesity are growing problems in developed and developing countries worldwide. It is generally accepted in the medical community that obesity is associated with negative health effects, such as an increased risk of diabetes, heart failure, high blood pressure and metabolic disorders. There is an ongoing need for means and methods which assist individuals in reducing weight or preventing weight gain.

It has been suggested in the art that certain herbs, teas or herbal active agents could support weight loss and control. However, the weight loss which can be achieved with such active agents is often not significant. It has generally not been proven that specific herbs, teas or herb components significantly mediate weight loss and reduce obesity over longer time periods.

In this respect, it has also been suggested that the consumption of green tea could help individuals to lose or control weight. However, the effect seems to be attributed rather to the filling of the stomach than to active ingredients which would reduce and control weight.

Various beverages or foods have been proposed or are commercially available which combine green tea with components allegedly having an effect on weight control.

CN 103891935 A discloses a beverage in the form of a health care and health-preserving tea, which is mainly composed of green tea and licorice. The inventors describe the tea as having efficacy of clearing heat, resolving phlegm, reducing weight, slimming, improving eyesight and calming the liver. However, no evidence of such effects is provided which is in line with general notion that significant effects on weight loss by consumption of beverages and foods are difficult to accomplish.

CN101658221A discloses a green tea beverage comprising Gynostemma pentaphyllum and Folium nelumbinis. The inventors assert that drinking the composition has various advantageous effects such as improving immunity, reducing blood fat or reducing weight, but no evidence for an effect is provided.

CN104247801A discloses a green tea beverage comprising Poria cocos, dutchmanspipe root and mulberry leaf. The inventors assert that drinking the composition has various advantageous effects such as skin whitening, toxin-expelling or reducing weight, or on artery sclerosis, but no evidence for an effect is provided.

US2010/0330189A1 relates to sustained-release beads providing active ingredients, such as caffeine, over an extended period of time after oral administration. It is suggested to include the beads into beverages in small amounts such as 0.1 weight-% such that they can be swallowed conveniently and slowly relase the active ingredient in the stomach. There is no suggestion to provide compositions related to weight loss. In contrast, the beads are dispersed in fructose sirup in the working examples.

EP 1 629 722 A1 discloses a pourable composition comprising a plurality of tasty gelled beads dispersed in a continuous aqueous phase. The beads contain 0.3-10 wt.%, preferably 0.5-4 wt.% of an anionic gelling polysaccharide selected from the group consisting of pectin, alginate and mixtures thereof. There is no suggestion to provide compositions related to weight loss. In contrast, a liquid feed composition comprising high levels of sucrose and glucose sirup is disclosed in the working examples.

In view of the above-described problems, there is an ongoing need for providing means, such as beverages or foods, for reducing and controlling weight of an individual.

### Problem underlying the invention

The problem underlying the invention is to provide a composition for oral consumption for reducing the weight of an individual. The composition shall assist the individual's desire to reduce weight, prevent weight gain or control his/her weight. The taste and general appearance of the composition should be pleasant or at least acceptable for the individual. The mode of consumption or administration should be easy and convenient. The composition should be easily available at relatively low costs. Overall, the composition should be applicable for long-time consumption at regular intervals or time points.

### Disclosure of the invention

Surprisingly, it was found that the problem underlying the invention is overcome by compositions and uses according to the claims. Further embodiments of the invention are outlined throughout the description.

Subject of the invention is a composition for oral administration comprising a beverage and beads distributed in the beverage, wherein the beads have an average diameter between 2 to 10 mm, the beads comprise more than 50% (w/w) dietary fibres and the amount of beads in the composition is between 1 and 20 wt.%, wherein the energy content of the composition is below 100 kJ/100 g.

The composition is for oral administration. Specifically, it is applicable for drinking in combination with eating the beads. The beads are chewed during or in between the drinking process. This is possible, because the relatively small beads are distributed in the beverage. When the composition is drunk, the beads are taken up whilst drinking and can be chewed whilst drinking or in between.

Overall, the composition and the beads which are mainly composed of dietary fibres have a relatively low energy content. It was found that consumption of such a low energy composition by drinking in combination with chewing of the beads has a significant effect on weight loss. It was found that the composition relieves hunger or appetite and that the effect is long-lasting. The effect seems to be correlated to epigenetic factors. It has been known in the art that drinking can alleviate a sense of hunger for a short time, because filling the stomach, associated with expansion of the stomach, activates mechanoreceptors in the stomach, thereby repressing a sense of hunger temporarily. However, this effect is only short-lived and does not have a significant effect on weight loss. Further, the chewing process is known to affect mechanoreceptors in the mouth, nose and pharynx area, which also represses a sense of hunger or appetite temporarily. Also this effect is short-lived and generally not considered to affect weight loss significantly, as observed for example when consuming chewing gums. However, the combined effect of stomach filling and chewing achieved with the specifically designed inventive composition, which has a low energy content, was found to induce weight loss or prevent weight gain significantly and permanently. Thus, the inventive composition is associated with a synergetic effect which could not be expected from the isolated components.

The composition comprises beads distributed in the beverage. The beads are solid. They maintain their shape and texture in the beverage at least for the time span during which the composition is consumed. Preferably, the beads are water-repellent. This implies that the beverage does not enter the interior of the beads and/or the beads are not soaked with or dissolved in the beverage at least during a time span required for consumption. For example, the beads should be stable and/or water-repellent in the beverage at least for 20 minutes, preferably up to 30 minutes, up to 40 minutes, up to 1 hour or up to 5 hours. Preferably, the beads are not gels.

The beads have an average diameter between 2 to 10 mm, preferably between 3 and 6 mm or between 4 and 5 mm. Preferably, all of the beads have a diameter between 2 to 10 mm, preferably between 3 and 6 mm or between 4 and 5 mm. When the beads are smaller than 2 mm, chewing is not as convenient any more for the consumers and they tend to be swallowed. When the average diameter is more than 10 mm, they may be too large for consumption whilst drinking in a desired amount and for convenient and uniform chewing. It was found that an optimal bead diameter for chewing in combination with drinking is between 4 to 5 mm. The diameter is determined in the largest diameter of the bead. The bead diameter can be adjusted in the production process, for example when the beads are produced by compression in moulds. If the beads should not be of a predetermined size, the bead average diameter can be determined by visual analysis, preferably by measuring the largest diameter of a significant number of beads, such as 100, 500 or 1,000 beads, and averaging.

The beads may have any shape applicable for uptake with a beverage through drinking and chewing. The shape is preferably round, for example spherical or ellipsoid. Preferably, the beads are spherical. Alternatively, they may have other typical shapes known from the production of dragees, tablets, pellets or the like.

The amount of beads in the composition is between 1 and 20 wt.%, preferably between 2 and 15 wt.% or between 4 and 10 wt.%. Overall, the ratio of the beverage to the beads should be adjusted such that the overall drinking and chewing time is similar, in other words that the individual can conveniently chew the beads whilst an in between drinking the beverage. In general, a large excess (w/w) of beverage is required to achieve a good balance between drinking the beverage and chewing the beads. The excess of beverage takes into consideration that the drinking process is usually faster than the chewing process.

The effect of the composition for reducing weight is supported by a relatively low food energy content of the composition. Thus, an individual consuming the composition does not take up a high amount of food energy, which would be contradictive to the purpose of weight loss.

Dietary fibres are the main component of the beads. Accordingly, the beads comprise more than 50% (w/w) dietary fibres. Preferably, they comprise more than 60%, more than 70%, or even more than 80% or more than 85% or more than 90% (w/w) dietary fibres. Preferably, the amount of dietary fibres is between 50% and 98%, more preferably between 80% and 95% (w/w).

As used herein, the term "dietary fibre" relates to carbohydrates, which are applicable in food, which are not digestible in humans plus lignin. Specifically, dietary fibres are carbohydrate polymers (polysaccharides), preferably with at least 10 monomeric units, which are not hydrolysed by the endogenous enzymes in the small intestine of humans. Since such fibres cannot be digested by intestinal enzymes, the energy cannot be taken used by the individual and the food energy content is low. Preferably, the dietary fibres are carbohydrates or derivatives thereof. As used herein, the term "derivatives" relates to chemically modified carbohydrates which are generally approved of or usable in food industry. The fibres may be natural, preferably from plants, or artificial, or post-treated natural fibres. The term "dietary fibres" encompasses "functional fibres". In a preferred embodiment, the fibres are natural fibres and/or derivatives of natural fibres. Preferably, the dietary fibre is approved of as a food component in the European Community or the U.S.A. The dietary fibres may be cellulose, alginic acids, arabinoxylans, inulin, chitins, pectins, resistant starch, resistant dextrins, and beta-glucans, or derivatives thereof.

In a preferred embodiment, the beads comprise dietary fibres obtained from seaweeds (algae), such as polyelectrolytes, such as agar, alginic acid, sodium alginate or carrageenan, or derivatives thereof. Fibers from seaweeds could be from Eucheuma, Gigartina and/or Chondrus. The fibres may also be natural gums obtained from plants, for example polyelectrolytes, such as gum arabic, or a derivative thereof.

Preferably, the polysaccharide derivative is an ether, preferably a C1 to C5 alkyl ether, more preferably a methyl, ethyl or propyl ether; or an ester, preferably of a C1 to C20 carboxylic acid, more preferably an acetylic acid ester; or substituted with carboxyalkyl groups having 1 to 5 C atoms, preferably carboxymethyl groups. Polysaccharide derivatives applicable as food additives are well known in the art. For example, they are described in "Food Polysaccharides and Their Applications", Alistair M. Stephen, Glyn O. Phillips, 2006 CRC Press.

In a preferred embodiment, the dietary fibres are not fermentable in humans. In this context, the term "fermentable" means that the dietary fibre is not digested by intestinal bacteria. Specifically, the carbohydrate chain is typically not cleaved. Dietary fibres which are not fermented have an extremely low food energy content.

In a preferred embodiment, the dietary fibres are cellulose fibres or derivatives thereof. Typically, cellulose and derivatives thereof are neither digested by intestinal enzymes nor fermented by intestinal bacteria. Thus, the food energy content of cellulose and derivatives thereof is practically about 0 kJ/mol. As used herein, the term "cellulose derivative" relates to cellulose which has been modified by a chemical reaction, and which is applicable and/or approved of for use in food. Preferably, the derivative is a cellulose ether, preferably a C1 to C5 alkyl ether, more preferably a methyl, ethyl or propyl ether; or an ester, preferably a C1 to C20 carboxylic acid ester, more preferably an acetylic acid ester; or substituted with carboxyalkyl groups having 1 to 5 C atoms, preferably with carboxymethyl groups. The cellulose ether and/or ester may be substituted at one, two or three hydroxyl groups of a glucose ring unit. Preferably, the cellulose derivative is selected from methylcellulose (MC), hydroxypropyl methylcellulose (HPMC), hydroxypropylcellulose (HPC), carboxymethylcellulose (CMC), ethylmethylcellulose (MEC) or microcrystalline cellulose (MCC). Cellulose derivatives, especially cellulose ethers, which are applicable as food additives are well known in the art. For example, they are described in "Cellulose and Cellulose Derivatives in the Food Industry: Fundamentals and Applications", 2015, Wiley-VCH Verlag GmbH & Co. KGaA, Tanja Wüstenberg (Editor). Preferably, the derivative is approved of for use in foods in the European Community or U.S.A at the priority date of this application.

The beads have a low energy content. Preferably, the energy content of the beads is below 500 kJ/100g, preferably below 400 kJ/100 g, more preferably below 300 kJ/100 g, most preferably below 200 kJ/100 g or even below 150 kJ/100 g. Such low energy contents may be adjusted by including high ratios of dietary fibres, which are not fermentable.

According to this application, the energy content of the beads is determined with a standard energy table for the main food components fat (37 kJ/g), ethanol (29 kJ/g), proteins (17 kJ/g), carbohydrates (17 kJ/g), organic acids (13 kJ/g), polyols (10 kJ/g) and dietary fibres (8kJ/g) with the modification that the energy content of non-digestible and non-fermentable fibres, such as cellulose derivatives, is set to 0 kJ/g. The energy content of other unknown ingredients can be determined calorimetrically.

The beverage (without the beads) should have a relatively low energy content. Preferably, the energy content of the beverage (without the beads) is below 25 kJ/100 g, preferably below 10 kJ/100 g, below 5 kJ/100 g, or even below 2 kJ/100, or about 0 kJ/100g. Such low energy contents can be achieved with water, tea or diluted juice. The energy content of the beverage could be calculated as described above for the beads. Alternatively, the energy content of the beverage could be determined calorimetrically.

The energy content of the composition is below 100 kJ/100 g, preferably below 50 kJ/100 g or below 25 kJ/100 g, more preferably below 20 kJ/100g or even below 15 kJ/100g or below 10 kJ/100g. Such low energy contents can be achieved with the beads and a low energy beverage as described above.

The low energy content of the composition, beads and beverage is advantageous for reducing food energy uptake when consuming the composition. Thus, the energy content of the composition supports the beneficial effect of the composition on weight loss achieved through drinking and chewing. Thereby, a sense of hunger or appetite can be supressed although the overall energy uptake is low.

The beverage may be selected from water, such as mineral water, tea, diluted fruit juice or low-energy soft drinks. In a preferred embodiment, the beverage is a natural beverage. Preferably, it is a tea, more preferably green tea. Green tea combines various advantageous properties. The energy content is about 2 kJ/100 g and thus very low. Green tea is generally recommended in the art for reducing a transient sense of appetite. Further, it is suspected to include active agents which may prevent obesity. Green tea is also generally accepted by individuals having a desire to increase their health and well-being. Thus, green tea can support the advantageous effects of the inventive composition.

In a preferred embodiment, the beads comprise at least one plant active agents, such as phytosterols, saponins, glycyrrhizin, liquiritin, liquiritigenin, oterin, orthosiphon or bromelin. Preferably, the plant active agent is preferably provided in the form of a plant extract. Preferably, the plant extract comprises active agents and/or is deprived of energy rich components of the plant, such as proteins, lipids and carbohydrates. In a preferred embodiment, the plant is selected from Glycyrrhiza glabra, Citrus aurantium (synephrine), wild yam (Dioscorea villosa), Mentha aquatica, Centella asiatica, Crataegus and Gingko, or fruits and vegetables, preferably pineapple (bromelain), mango or papaya.

In a preferred embodiment, the plant active agent supports weight loss. In a preferred embodiment, the beads comprise an extract from Glycyrrhiza glabra, which is preferably enriched for glycyrrhizin. The active ingredient glycyrrhizin may have a positive effect on weight loss and thus could support the effect of the composition.

In a preferred embodiment, the composition does not comprise sugars for sweetening, i.e. saccharide sweeteners. Preferably, such sugar sweeteners were not included as additives. Preferably, the composition does not comprise monosaccharides and/or disaccharides. Preferably, it does not comprise glucose, lactose or fructose. This means that the monosaccharides are not present in significant amounts. Insignificant amounts may be present in the composition because they are contained in additives, such as extracts from natural sources such as licorice. Preferably, the composition comprises less than 1% (w/w), preferably less than 0.5% (w/w) or less than 0.2% (w/w), sugars which are selected from monosaccharides and disaccharides. Preferably, the beads and/or the beverage comprise less than 1% (w/w), preferably less than 0.5% (w/w) or less than 0.2% (w/w), sugars which are selected from monosaccharides and disaccharides. Additives should be selected which do not increase the energy content of the composition significantly. i.e. which is not detrimental to the desired effect on weight loss. If at all, additives which do not have low energy content should only be added in small amounts. Additives should be selected such that the overall energy content is kept low. Low molecular weight saccharides, especially mono- and disaccharides, have a high energy content and should be avoided. Preferably, the composition and/or the beads are sweetened with other sweeteners, such as stevia.

In a preferred embodiment, the beads comprise at least 70% dietary fibres, which are cellulose derivatives, especially methyl cellulose and/or hydroxypropyl methylcellulose, and a liquorice extract, preferably in combination with green tea as the beverage. In a preferred embodiment, the beverage is green tea and the beads comprise glycyrrhizin and methylcellulose and/or hydroxypropyl methylcellulose. Glycyrrhizin and green tea may have positive effects on weight loss and thus may support the effect of the composition, whereas cellulose derivatives are especially applicable as low energy polymers.

In a preferred embodiment, the beads comprise at least one additional additives, which are not dietary fibres or plant active agents. In principle, any additive can be used which is applicable in the technical field of production of dragees, food pellets or tablets. When used in larger amounts, the additive should have an adequate energy content. The additive may confer desired properties to the beads or improve production and stability of the beads. Preferably, the additive is selected from sweeteners, such as stevia, binders, coatings, food supplements, such as chromium polynicotinate, antiadherents, colorants, disintegrants, flavours, glidants, lubricants, preservatives, sorbents and hydrophobic substances. Since sweeteners, flavours, colorants, plant extracts and the like can be combined easily with the dietary fibres, the composition can be provided with a pleasant taste and appearance. This is advantageous for supporting regular consumption by an individual over extended time periods.

In a preferred embodiment, the beads are water-repellent. This means that water drips off the beads when brought into contact with them and/or does not penetrate the beads. For example, additives can render the beads water-repellent. Preferably, these are hydrophobic substances, such as lipids, fats and/or waxes, preferably mono- and diglyceride fatty acid esters, especially esters from tartaric acid. Alternatively, the beads may be coated with a water-repellant coating. The beads may comprise any known coatings used in food or tablet technology. Preferably, the coating comprises a polymer, especially a polysaccharide, such as a cellulose derivative, such as hydromellose-phthalate (HPMC-P), or shellack. The coating can be applied by known methods, such as spray-coating.

The beads can be produced by known technical processes from food or pharmaceutical industry for producing similar small beads, such as dragees, tablets, pellets, chewing gums or the like. The beads can be produced by compressing the components. Often, dietary fibre polysaccharides, which are the main component of the beads, can be compressed into relatively stable beads. Compression and stability of the beads may be improved by moisture in the formulation. If required, binders may be added which improve internal strength of the beads. Some dietary fibres, such as methyl cellulose, may even function as binders.

If desired, additives may also be included into the beverage, such as flavours, colorants or active agents. In a preferred embodiment, the composition comprises epigallochatingelate, an active agent from green tea.

In principle, the beads are adapted such that they are chewed up within about 10 seconds to 200 seconds, preferably between about 20 to 90 seconds, often between about 30 to 60 seconds. Thus, the chewing process requires more effort than compared to most regular foods, which is advantageous for reducing overall energy uptake.

Further described herein is a device (a system) for storing and/or for preparing the composition for oral administration, the device comprising the beverage and the beads for distribution in the beverage as described above. The ratio of the beads and the beverage in the device is adapted such that the composition comprises between 1 and 20 wt.% beads when all the beads in the device are mixed with the beverage in the device. Preferably, the composition does not comprise monosaccharides. This means that neither the beads nor the beverage comprises monosaccharides. The device comprises containers in which the beverage and the beads are maintained separately from each other. Preferably, the containers adjoin each other.

Such a device (a system) can be provided to a consumer who can mix the beverage with the beads before consumption. Thereby, the texture and properties of the beads can be maintained for longer time periods. In a preferred embodiment, the device is a beverage container for drinking, such as a cup or bottle, with a compartment comprising the beads attached thereto. Preferably, the device comprises removable means for separating the beads from the beverage. For example, the beads could be embedded in a compartment above a cup, which is separated from the beverage by a thin barrier, such as a paper or film. The device may comprise a drinking aid, such as a straw. The straw has a sufficient width for taking up the beverage and the beads through the straw. It was found that normal consumption of such a beverage is possible through a straw without problems regarding swallowing of beads or the like. The device could be adapted such that the barrier between the beverage and beads is destroyed when the straw is entered into the beverage such that the beads are released into the beverage. Preferably, the device comprises between 200 and 1000 ml beverage, preferably between 250 and 750 or between 200 and 500 ml.

Another subject of the invention is the non-therapeutic use of a composition as described above for reducing the weight of an individual and/or preventing weight gain by oral administration. Further described herein is a method for reducing the weight of an individual and/or for preventing weight gain, comprising a step of administering the composition to an individual.

In a preferred embodiment, the use is for consumption of a total daily amount of 200 to 2000 ml, preferably 200 to 1000 ml, or 200 to 600 ml, and/or for consumption 1 to 5 times per day, preferably once or twice per day. Preferably, the use is for long term consumption, preferably for at least 10, 20 or 50 days. Preferably, the use is for consumption at the late afternoon or in the evening, for example after 4 pm or after 6 pm. In a specific embodiment, the use is for consumption by women, which are preferably aged above 40 or above 50. In a specific embodiment, the consumers have experienced weight gain before administration.

In another embodiment, the use is for women during or after menopause. Preferably, these women have experienced weight gain during or after menopause.

In a preferred embodiment, the use is in combination with a diet. In this case, the use is for supporting the diet. Thus, the composition is consumed whilst the overall uptake of food energy is reduced. Specifically, the use is for supporting food abstinence after consumption. Preferably, after having consumed the composition, the individual does not consume food any more the same day. It was found that it is easier for individuals to be abstinent from food after consumption of the composition than without consuming the composition.

Preferably, a dosage of a composition is consumed during a time period between 10 and 30 minutes, preferably about 15 minutes. The amount of beads in the composition should be adjusted such that sufficient beads are available for continuous chewing throughout this time period.

The beads as described above are used for preparing the composition for oral administration or the device as described above. Preferably, the beads are a plurality of beads, for example an amount of at least 5 or at least 10 g.

In a preferred embodiment, the beads are not slow-release beads, especially not as those disclosed in US2010/330189A1. Since the beads are chewed, and since the effect on weight loss does not rely on active agents included, it is not necessary to design the beads such that an active ingredient is slowly released. In a specific embodiment, the beads do not comprise an active ingredient for reducing weight. Without being bound to theory, it is assumed that the effect of the composition can be attributed to the physical combination of the beverage with chewable beads. In a preferred embodiment, the beverage does not comprise a thickening agent.

Exemplified embodiments of the invention and aspects of the invention are shown in the figures.
Figure 1 shows beads for use in the composition.
Figure 2 shows a composition, in which beads are distributed in a beverage.

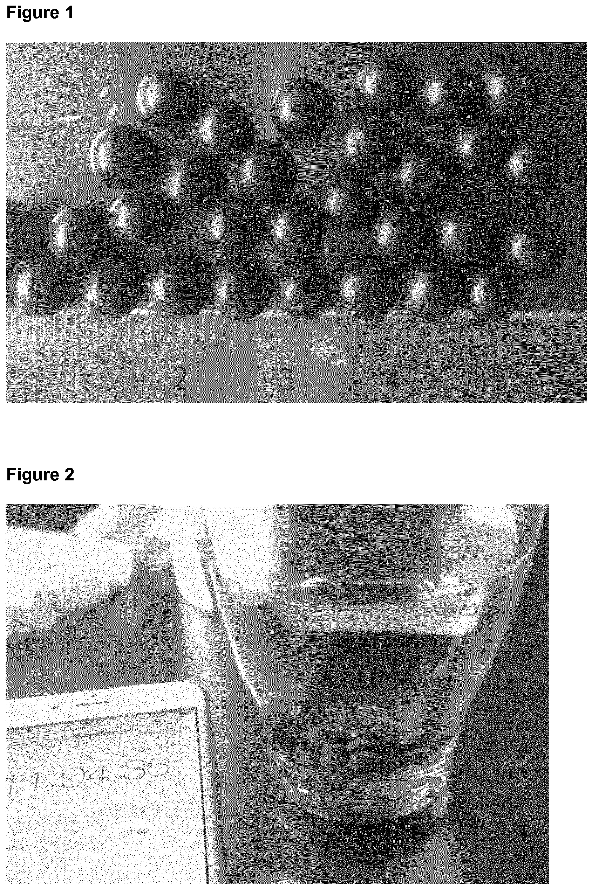

In Fig. 1, an advantageous and typical embodiment of the beads is shown. The beads have regular spherical size and average diameters of about 4 to 5 mm. This size is appropriate for taking up the beads whilst drinking or sucking up the beads with the beverage with a straw. The uniform and smooth surface is pleasant for the consumer.

In Fig. 2, an inventive composition with beads distributed in a beverage is shown. The beads are heavier than the beverage and accumulate on the bottom of the cup. However, the beads could also be construed to float in the beverage or on top of the beverage by adjusting the content accordingly, for example if the beads are porous. The accumulation on the ground of the beverage is advantageous for sucking with a straw.

The inventive composition solves the problem underlying the invention. In trials with individuals who consumed the composition over extended periods of time, a significant effect on weight loss was observed. Further, it was found on a physiological level that the composition has an epigenetic effect. Physiological markers were found to be affected which control and influence weight obesity. Specifically, it was found that administration of the composition regularly over a period of several weeks lowers the level of proneurotensin in the blood. Proneurotensin is a known biomarker for weight control. The level of neurotensin is significantly determined by animal fats and sugars. Thus, the reduced proneurotensin level in the blood provides evidence that the inventive composition has an epigenetic effect on the consumers.

Thus, the composition provides a novel and advantageous approach for weight loss of individuals. The approach is special because of the purposeful combination of physical filling of the stomach and activation of mechanoreceptors in the mouth area through chewing, with the specifically adapted beads and composition. The composition can be varied widely in taste and appearance and thus can be pleasant for the user. The chewing of the beads can also be pleasant for the consumer, and in this regard can be similar to consumption of chewing gum. In addition, active ingredients may be included which support appetite reduction and weight loss.

### Examples

### Example 1: Preparation of beads

Beads were prepared based on various dietary fibres as the main component. The dietary fibres were mostly methylcellulose and hydroxypropyl methylcellulose. Further, fibres from seaweeds such as Eucheuma, Gigartina and Chondrus were included. Such dietary fibres are known and commonly used in food technology. Stevia was included as a sweetener. Various plant extracts were included which have a pleasant taste or appearance or which have been described in the art as improving the well-being of the consumer. These comprised plant active agents such as glycyrrhizin, phytosterole, saponins, liquiritin and liquiritigenin. Wild yam contains high amounts of steroidal saponins. The Glycyrrhiza glabra (licorice) extract was enriched in active agents such as glycyrrhizin, phytosterole, saponins, liquiritin and liquiritigenin. As examples, beads were prepared with the following base formulations:
Formulation A: methylcellulose, hydroxypropyl methylcellulose, Algae mixture (Eucheuma, Gigartina, Chondrus), stevia, Glycyrrhiza glabra extract, pinapple extract (bromelain), Citrus aurantium (synephrine) extract.
Formulation B: methylcellulose, hydroxypropyl methylcellulose, Algae mixture (Eucheuma, Gigartina, Chondrus), stevia, wild yam (Dioscorea villosa) extract, Glycyrrhiza glabra extract.
Formulation C: methylcellulose, hydroxypropyl methylcellulose, Algae mixture (Eucheuma, Gigartina, Chondrus), stevia, Glycyrrhiza glabra extract, chromium polynicotinate, Mentha aquatica extract.
Formulation D: methylcellulose, hydroxypropyl methylcellulose, Algae mixture (Eucheuma, Gigartina, Chondrus), stevia, Glycyrrhiza glabra extract.

Further additives can be included into the formulation for conferring desired physical properties to the beads. Lipids, such as tartaric acid esters of fatty acid glycerides, were added as binders and to render the beads water repellant. The ingredients were mixed, kneaded and compressed into spherical beads. The beads were coated with hydromellose-phthalate (HPMC-P) by spray coating for protection and increasing water repellant properties. A formulation E with all additives is shown in table 1.

**Table 1: Formulation E**

| **Ingredient** | **mg** | **%(w/w)** |
|---|---|---|
| Hydroxypropyl-Methylcellulose | 16,800 | 84 |
| Alginic acid coated with HPMC-P | 1,300 | 6.5 |
| Tartaric acid esters of mono-and diglycerides of fatty acids | 950 | 4.8 |
| Stevia | 850 | 4.2 |
| Glycyrrhiza glabra | 100 | 0.5 |
| Total | 20,000 | 100 |
| Spray coating: Hypromellose-Phthalat (HPMC-P) | not determined | |

The spherical beads had a diameter of about 4 to 5 mm (Figure 1). The food energy content of 20 g beads, which relates to a typical single dosage, was determined as 25 kJ (corresponding to 125 kJ/100 g beads; based on an energy content of non-fermentable cellulose derivatives of 0 kJ/100g). When starting chewing the beads, the texture was slightly resilient and roughly comparable to chewing gum. It took about 30 to 60 seconds for the beads to be chewed up in the mouth.

### Example 2: Preparation of composition

Green tea containing about 20 mg ±5 % epigallochatingelate per serving (300 ml) was used as the beverage for preparing the composition. 20 g of beads prepared according to example 1 were poured into 300 g cold green tea. The beads were water repellent and could be dispersed in the tea. The beads sank to the bottom of the cup as shown in figure 2. The beads did not soften or change texture and dissolve within about 40 minutes after contact with water. It took about 120 minutes for the beads to dissolve completely. Since the beads are chewed, drinking took longer than for a normal drink without beads. A typical drinking time for 300 g green tea with 20 g beads was about 5 to 20 minutes. The energy contents of inventive and some comparative compositions are summarized in table 2 below.

**Table 2: Energy densities of inventive and comparative compositions**

| **Composition** | **kJ** | **kJ/100g** |
|---|---|---|
| 20 g Beads (formulation E) | 25 | 125 |
| 300ml tea and 20 g beads (formulation B) | 41.8 | 13.1 |
| 300ml tea and 20 g beads (formulation D) | 25.7 | 8.0 |
| 300 ml green tea with 10 g licorice and 10 g wild yam | 1291 | 403.4 |
| 300 ml Citrus Green Tea (trademark Nestea, Nestle, CH) | 240 | 80 |
| 300 ml Green tea ice tea (trademark Lipton, Unilever, NL) | 237 | 79 |

The results demonstrate that the energy content of the beads and the composition is very low, especially when compared to commercially available green tea beverages or natural products.

### Example 3: Effect of composition

The composition was administered to individuals in order to assess the effect on weight and epigenetic factors. Trials were carried out with a composition as described in example 2 based on 300 ml green tea and 20 g beads of composition D comprising 850 mg stevia and 100 mg Glycyrrhiza glabra. The composition was administered to seven individuals once per day over time periods between 40 and 51 days. The individuals were women aged between 51 and 54 years which had previously experienced weight gain between 4 to 9 kg since menopause and were not subject to hormone therapy. The individuals had tried to reduce weight in the past by food abstinence after 4 p.m. daily, but had not been successful. Thus, they agreed to try administration of the composition once after 4 p.m. daily. The subjects were allowed to select the time of administration after 4 p.m. depending on a sensation of hunger. Further, the individuals were abstinent from food after 4 p.m. daily. The subjects were instructed to consume 500 ml green tea slowly over a period of 15 minutes. At the same time, the subjects chewed beads. The subjects were instructed to consume a number of beads appropriate for a normal chewing, to prolong chewing as long as possible and to drink the green tea simultaneously.

As a result, five of the seven subjects were able to consume the composition daily under the predetermined conditions. Two patients were only able to consume the composition under the predetermined conditions two to five times per week. An overview of the conditions and results is provided in the following table. For the five subjects who consumed the composition daily, a weekly weight loss between 500 g and 1 kg per week was observed. All women reported that consumption of the composition under the predetermined conditions reduced a feeling of hunger significantly. Thus, the women were able to continue the consumption over extended time periods, whilst food abstinence after 4 pm had failed before without the inventive composition. Side effects, such as stomach pain or digestion problems were not observed. The total weight loss observed was between 2.4 and 5.1 kg for all subjects with an average of 3.9 kg. A summary of conditions and results is provided in table 3.

In order to assess an effect on the physiological level, the concentration of proneurotensin was determined from blood of the subjects at the beginning and end of the trial. Proneurotensin is a known biomarker for weight control. Neurotensin is formed in N-cells of the small intestine and in the central nervous system. The concentration of neurotensin is greatly affected by animal fats and sugars. By daily administration of the composition, the level of proneurotensin could be lowered for all subjects at an average of 13 pm/l, which is roughly about 10%. The results are also summarized in table 3.

**Table 3: Conditions and results of trials according to example 3 (diff = difference; Proneu = proneurotensin).**

| **Subject** | **Age** | **Weight start** | **Weight end** | **Weight diff** | **Proneu start** | **Proneu end** | **Proneu diff** | **Time** |
|---|---|---|---|---|---|---|---|---|
| [number] | [years] | [kg] | [kg] | [kg] | [pmol/l] | [pmol/l] | [pmol/l] | [days] |
| 1 | 51 | 72.4 | 67.3 | -5.1 | 120 | 118 | -12 | 43 |
| 2 | 51 | 85.2 | 81.8 | -3.4 | 143 | 119 | -24 | 40 |
| 3 | 55 | 77.6 | 72.9 | -4.7 | 139 | 119 | -20 | 46 |
| 4 | 51 | 89.0 | 86.6 | -2.4 | 152 | 140 | -12 | 40 |
| 5 | 54 | 81.9 | 79.1 | -2.8 | 139 | 130 | -9 | 51 |
| 6 | 53 | 75.5 | 71.2 | -4.3 | 131 | 125 | -6 | 46 |
| 7 | 51 | 68.6 | 64.3 | -4.3 | 149 | 140 | -9 | 48 |
| average | | | | -3.9 | | | -13 | |

Overall, the results demonstrate a significant effect of administration of the composition on weight loss for all subjects who participated in the trial, which could not be achieved simply by food abstinence.

### Example 4: Effect of comparative composition without beads

In order to exclude that the positive results of example 3 are obtained because of active ingredients in the composition, a further trial was carried out. A comparable formulation F was prepared with the active ingredients of composition D of example 3. However, the composition was not provided in the form of a beverage with chewable beads. Instead, formulation F was a beverage for drinking prepared by dissolving the ingredients in the green tea.

### Formulation F (32 kJ per 500 ml):

500 ml green tea contained 100 mg Glycyrrhiza glabra, 850 mg Stevia, 35 g algea mixture (Eucheuma, Gigartina, Chondrus).

The individuals consumed formulation F once per day over time periods between 40 and 49 days. The individuals were 11 women aged between 51 and 54 years which had previously experienced weight gain between 4 to 9 kg since menopause and were not subject to hormone therapy. The individuals had tried to reduce weight in the past by food abstinence after 4 p.m. daily, but had not been successful. Thus, they agreed to drink 500 ml of formulation F once a day after 4 p.m. The subjects were allowed to select the time of administration after 4 p.m. depending on a sensation of hunger. Further, the individuals were abstinent from food after 4 p.m. daily. The subjects were instructed to consume the 500 ml formation F slowly over a period of 15 minutes.

The results are summarized in table 4 below. An average weight gain of 0.3 kg was observed for the individuals. Otherwise, no significant change of other body markers was observed. There were no significant changes in proneurotensin levels.

**Table 4: Conditions and results of trials according to example 4 (diff = difference; Proneu = proneurotensin).**

| **Subject** | **Age** | **Weight start** | **Weight end** | **Weight diff** | **Proneu start** | **Proneu end** | **Proneu diff** | **Time period** |
|---|---|---|---|---|---|---|---|---|
| [number] | [years] | [kg] | [kg] | [kg] | [pmol/l] | [pmol/l] | [pmol/l] | [days] |
| 1 | 51 | 78.6 | 78.1 | -0.5 | 118 | 117 | -1 | 41 |
| 2 | 53 | 61.8 | 62.3 | +0.5 | 136 | 128 | -8 | 45 |
| 3 | 53 | 55.6 | 54.9 | -0.7 | 108 | 115 | -7 | 48 |
| 4 | 52 | 59.6 | 62.1 | +1.5 | 118 | 117 | -1 | 47 |
| 5 | 54 | 73.4 | 73.6 | +0.2 | 142 | 154 | +12 | 49 |
| 6 | 52 | 82.3 | 85.8 | +3.5 | 123 | 121 | -2 | 43 |
| 7 | 54 | 61.3 | 60.2 | -0.9 | 106 | 117 | +11 | 47 |
| 8 | 53 | 64.2 | 63.5 | -0.7 | 134 | 131 | -3 | 48 |
| 9 | 52 | 84.6 | 84.3 | -0.3 | 147 | 149 | +2 | 44 |
| 10 | 51 | 53.5 | 54.6 | +1.1 | 108 | 114 | +6 | 40 |
| 11 | 52 | 58.4 | 57.6 | -0.8 | 124 | 112 | -10 | 40 |
| average | | | | +0.3 | | | 0 | |

The results demonstrate that simply drinking green tea with dissolved dietary fibers and Glycyrrhiza glabra, stevia and algea does not stimulate weight loss and does not improve neurotensin (proneurotensin) levels. Example 3 and comparative example 4 taken together provide evidence that the effects of the inventive composition on weight loss and proneurotensin levels result from the dosage form as a beverage comprising chewable beads.

## Claims

1. A composition for oral administration comprising a beverage and beads distributed in the beverage, wherein the beads have an average diameter between 2 to 10 mm, comprise more than 50% (w/w) dietary fibres and wherein the amount of beads in the composition is between 1 and 20 wt.%, wherein the energy content of the composition is below 100 kJ/100 g.

2. The composition of claim 1, wherein the dietary fibres are cellulose fibres or derivatives thereof.

3. The composition according to at least one of the preceding claims, wherein the dietary fibres are not fermentable in humans.

4. The composition according to at least one of the preceding claims, wherein the energy content of the beads is below 500 kJ/100 g, preferably below 150 kJ/100 g.

5. The composition according to at least one of the preceding claims, wherein the beverage without the beads comprises less than 1% (w/w) carbohydrates.

6. The composition according to at least one of the preceding claims, wherein the beverage is green tea.

7. The composition according to at least one of the preceding claims, wherein the beads comprise at least one plant extract and/or plant active agents, wherein the plant is preferably selected from Glycyrrhiza glabra, Citrus aurantium (synephrine), wild yam (Dioscorea villosa), Mentha aquatic, Centella asiatica and Gingko, or fruits and vegetables, preferably selected from pineapple (bromelain), mango or papaya.

8. The composition according to at least one of the preceding claims, wherein the beads comprise at least one additive, which is preferably selected from sweeteners, such as stevia; food supplements, such as chromium polynicotinate, antiadherents, binders, coatings, colorants, disintegrants, flavours, glidants, lubricants, preservatives, sorbents and hydrophobic substances.

9. The composition according to any of the preceding claims wherein the beverage is green tea and wherein the beads comprise methylcellulose and/or hydroxypropyl methylcellulose and glycyrrhizin.

10. The composition according to at least one of the preceding claims, wherein the beads are round, preferably spherical or ellipsoidal, and/or wherein the beads comprise a coating, and/or wherein the beads are water-repellent.

11. The composition according to at least one of the preceding claims, wherein the composition comprises less than 1% (w/w) sugars which are selected from monosaccharides and disaccharides.

12. Non-therapeutic use of a composition of at least one of the preceding claims for reducing the weight of an individual and/or for preventing weight gain by oral administration.

13. The non-therapeutic use of claim 12 for consumption of a total daily amount of 200 to 2000 ml and/or for consumption once or twice a day.

## Patentansprüche

1. Zusammensetzung zur oralen Verabreichung, umfassend ein Getränk und in dem Getränk verteilte Kügelchen, wobei die Kügelchen einen durchschnittlichen Durchmesser zwischen 2 und 10 mm haben, mehr als 50 Gew.-% diätetische Fasern umfassen und wobei die Menge an Kügelchen in der Zusammensetzung zwischen 1 und 20 Gew.-% liegt, wobei der Energiegehalt der Zusammensetzung unter 100 kJ/100 g liegt.

2. Zusammensetzung nach Anspruch 1, wobei die diätetischen Fasern Cellulosefasern oder Derivate davon sind.

3. Zusammensetzung nach mindestens einem der vorhergehenden Ansprüche, wobei die diätetischen Fasern im Menschen nicht fermentierbar sind.

4. Zusammensetzung nach mindestens einem der vorangehenden Ansprüche, wobei der Energiegehalt der Kügelchen unter 500 kJ/100 g, vorzugsweise unter 150 kJ/100 g liegt.

5. Zusammensetzung nach mindestens einem der vorhergehenden Ansprüche, wobei das Getränk ohne die Kügelchen weniger als 1 Gew.-% Kohlenhydrate umfasst.

6. Zusammensetzung nach mindestens einem der vorhergehenden Ansprüche, wobei das Getränk grüner Tee ist.

7. Zusammensetzung nach mindestens einem der vorhergehenden Ansprüche, wobei die Kügelchen mindestens einen Extrakt einer Pflanze und/oder Wirkstoffe einer Pflanze umfassen, wobei die Pflanze vorzugsweise ausgewählt ist aus Glycyrrhiza glabra, Citrus aurantium (Synephrin), wildem Yam (Dioscorea villosa), Mentha aquatic, Centella asiatica und Gingko, oder Obst und Gemüse, vorzugsweise ausgewählt aus Ananas (Bromelain), Mango oder Papaya.

8. Zusammensetzung nach mindestens einem der vorhergehenden Ansprüche, wobei die Kügelchen mindestens einen Zusatzstoff umfassen, der vorzugsweise ausgewählt ist aus Süßungsmitteln, wie Stevia, Nahrungsergänzungsmitteln, wie Chrompolynikotinat, Antiadhäsionsmitteln, Bindemitteln, Beschichtungen, Farbstoffen, Sprengmitteln, Aromen, Gleitmitteln, Schmiermitteln, Konservierungsmitteln, Sorbentien und hydrophoben Substanzen.

9. Zusammensetzung nach einem der vorhergehenden Ansprüche, wobei das Getränk grüner Tee ist und wobei die Kügelchen Methylcellulose und/oder Hydroxypropylmethylcellulose und Glycyrrhizin umfassen.

10. Zusammensetzung nach mindestens einem der vorhergehenden Ansprüche, wobei die Kügelchen rund sind, vorzugsweise kugelförmig oder ellipsenförmig, und/oder wobei die Kügelchen eine Beschichtung umfassen, und/oder wobei die Kügelchen wasserabweisend sind.

11. Zusammensetzung nach mindestens einem der vorhergehenden Ansprüche, wobei die Zusammensetzung weniger als 1 Gew.-% Zucker umfasst, die aus Monosacchariden und Disacchariden ausgewählt sind.

12. Nicht-therapeutische Verwendung einer Zusammensetzung nach mindestens einem der vorhergehenden Ansprüche zur Gewichtsreduktion eines Individuums und/oder zur Verhinderung einer Gewichtszunahme durch orale Verabreichung.

13. Nicht-therapeutische Verwendung nach Anspruch 12 zur Einnahme einer täglichen Gesamtmenge von 200 bis 2000 ml und/oder zur ein- oder zweimaligen Einnahme pro Tag.

## Revendications

1. Composition pour une administration orale comprenant une boisson et des billes distribuées dans la boisson, les billes possédant un diamètre moyen compris entre 2 et 10 mm, comprenant plus de 50 % (p/p) de fibres alimentaires et la quantité de billes dans la composition étant comprise entre 1 et 20 % en poids, la teneur en énergie de la composition étant inférieure à 100 kJ/100 g.

2. Composition selon la revendication 1, les fibres alimentaires étant des fibres de cellulose ou des dérivés correspondants.

3. Composition selon au moins l'une des revendications précédentes, les fibres alimentaires n'étant pas fermentescibles chez des êtres humains.

4. Composition selon au moins l'une des revendications précédentes, la teneur en énergie des billes étant inférieure à 500 kJ/100 g, préférablement inférieure à 150 kJ/100 g.

5. Composition selon au moins l'une des revendications précédentes, la boisson sans les billes comprenant moins de 1 % (p/p) de glucides.

6. Composition selon au moins l'une des revendications précédentes, la boisson étant du thé vert.

7. Composition selon au moins l'une des revendications précédentes, les billes comprenant au moins un extrait végétal et/ou des agents actifs végétaux, le végétal étant préférablement choisi parmi Glycyrrhiza glabra, Citrus aurantium (synéphrine), l'igname sauvage (Dioscorea villosa), la menthe aquatique, Centella asiatica et Gingko, ou des fruits et des légumes, préférablement choisis parmi l'ananas (bromélaïne), la mangue ou la papaye.

8. Composition selon au moins l'une des revendications précédentes, les billes comprenant au moins un additif, qui est préférablement choisi parmi des édulcorants, tels que le stevia ; des suppléments alimentaires, tels le polynicotinate de chrome, des agents antiadhérents, des liants, des revêtements, des colorants, des désintégrants, des arômes, des agents glissants, des lubrifiants, des conservateurs, des sorbants et des substances hydrophobes.

9. Composition selon l'une quelconque des revendications précédentes, la boisson étant du thé vert et les billes comprenant une méthylcellulose et/ou une hydroxypropylméthylcellulose et de la glycyrrhizine.

10. Composition selon au moins l'une des revendications précédentes, les billes étant arrondies, préférablement sphériques ou ellipsoïdales, et/ou les billes comprenant un revêtement, et/ou les billes étant hydrofuges.

11. Composition selon au moins l'une des revendications précédentes, la composition comprenant moins de 1 % (p/p) de sucres qui sont choisis parmi des monosaccharides et des disaccharides.

12. Utilisation non thérapeutique d'une composition selon au moins l'une des revendications précédentes pour la réduction du poids d'un individu et/ou pour la prévention d'un gain de poids par administration orale.

13. Utilisation non thérapeutique selon la revendication 12 pour la consommation d'une quantité quotidienne totale de 200 à 2 000 ml et/ou pour une consommation une ou deux fois par jour.
